(19) Europäisches Patentamt
European Patent Office
Office européen des brevets

(11) Publication number: **0 182 669 A1**

# (12) EUROPEAN PATENT APPLICATION

(21) Application number: **85308489.5**

(22) Date of filing: **21.11.85**

(51) Int. Cl.[4]: **C 07 C 93/04**

(30) Priority: **21.11.84 US 673647**

(43) Date of publication of application: **28.05.86**
**Bulletin 86/22**

(84) Designated Contracting States: **BE DE FR GB IT NL**

(71) Applicant: **ATLANTIC RICHFIELD COMPANY, 515 South Flower Street, Los Angeles California 90071 (US)**

(72) Inventor: **Kesling, Haven S., Jr., 248 Friendship Road, Drexel Hill, Pennsylvania 19026 (US)**
Inventor: **Cooper, Charles F., 26 Wistar Road, Paoli Pennsylvania 19301 (US)**

(74) Representative: **Cropp, John Anthony David et al, MATHYS & SQUIRE 10 Fleet Street, London, EC4Y 1AY (GB)**

## (54) Quaternary Ammonium compounds.

(57) An alkoxylated quaternary ammonium compound of the formula

$$\left[ RO(R_1)_xCH_2\overset{\displaystyle R_2 \atop |}{C}H\overset{\oplus}{N}R_3R_4R_6 \right] A^{\ominus}$$

or a mixture of such compounds, wherein R is a straight or branched chain alkyl group having from 1 to 11 carbon atoms, a cyclic alkyl group having from 5 to 10 carbon atoms, an aryl group having up to 12 carbon atoms or an aralkyl or alkaryl group having up to 18 carbon atoms, each $R_1$ unit has the formula:

$$\left( CH_2\overset{\displaystyle R_5 \atop |}{C}HO \right)$$

wherein in each unit $R_5$ is independently selected from hydrogen and straight and branched chain alkyl groups having from 1 to 12 carbon atoms, x is an integer of from 0 to 40, $R_2$ is hydrogen or a straight or branched chain alkyl group having from 1 to 12 carbon atoms, $R_3$ and $R_4$ are each independently selected from straight and branched chain alkyl groups having from 1 to 12 carbon atoms, cyclic alkyl groups having from 5 to 10 carbon atoms, 1 to 4 carbon atom alkyl substituted or unsustituted benzyl groups and allyl, $R_6$ is a straight or branched chain alkyl group having from 1 to 18 carbon atoms, a cyclic alkyl group having from 5 to 10 carbon atoms, a 1 to 4 carbon atom substituted or unsubstituted benzyl group or an allyl group and A is an anion.

EP 0 182 669 A1

The present invention relates to novel alkoxylated quaternary ammonium compounds and a process for preparing them.

In our co-pending European patent application no. 85307815.2 filed 29th. October , 1985 entitled, Alkoxylated Tertiary Amine Compounds and incorporated herein by reference, there is disclosed novel alkoxylated tertiary amine compounds. The amine compounds of the above mentioned application are employed to prepare the novel quaternary ammonium compounds of the instant invention. The quaternary ammonium compounds of the invention are useful, for example, as fabric softeners, in the preparation of organophilic clays for drilling muds and high quality rheology control, polymer antistats and plastic additives, as biocides (in personal care products, oil patch, paper mill, industrial cleaners and sanitizers, swimming pool additives and in cosmetics), as corrosion inhibitors, as textile antistats, softeners, etc., as demulsifiers, in ore flotation processes and hydrometallurgy, as fertilizer conditioners, as lubricants and fuel additives and in soil stabilization. These and many other applications are known to those skilled in the art. Each of the above noted uses have many specific smaller uses. For example, organophilic clays are employed in hundreds of small and large applications where rheological control is important. Such applications include drilling fluids, foundry sands, leakage and spill control, paints and coatings, polymer fillers, sealants, cosmetics, drugs,

rubber applications and many more.

Quaternary ammonium compounds including ethoxylated and propoxylated compounds of various structures are known as may be shown for example in U. S. Patent Numbers 3,872,138, 4,134,970 and 4,139,477. The alkoxylated quaternary ammonium compounds of the instant invention have many advantages over the prior art commercially available quaternary ammonium compounds. To name a few, the instant alkoxylated quaternary ammonium compounds have good hydrocarbon solubility, excellent surface activity, detergency on oil based soils, cold water dispersibility, excellent compatibility, with anionic surfactants, low foam and excellent biostat and antistat characteristics.

It is an object of this invention to provide a novel class of alkoxylated quaternary ammonium compounds including a method for their preparation.

These and other objects and advantages of this invention will become apparent from the description of the invention which follows and from the claims.

## DESCRIPTION OF THE INVENTION

The present invention provides novel alkoxylated quaternary ammonium compounds of the general formula:

$$\left[ RO(R_1)_xCH_2\overset{\overset{R_2}{|}}{C}H\overset{+}{N}\ R_3R_4R_6 \right] A^{\ominus}$$

wherein R is a straight or branched chain alkyl group having from 1 to 11, preferably from 6 to 10 carbon atoms, a cyclic alkyl group having from 5 to 10 carbon atoms, preferably from 5 to 6 carbon atoms, an aryl group having up to 12 carbon atoms, preferably 6 to 10 carbon atoms, or

an aralkyl or alkaryl group having up to 18 carbon atoms, preferably 7 to 12 carbon atoms, $R_1$ is a single unit or a series of units of the formula:

$$\left(CH_2\overset{R_5}{\overset{|}{C}}HO\right)_x$$

wherein in each unit $R_5$ is independently selected from the group consisting of hydrogen and a straight or branched chain alkyl group having from 1 to 12 carbon atoms, preferably 1 to 4 carbon atoms, and X is an integer of from 0 to 40, preferably from 3 to 20, $R_2$ is hydrogen or a straight or branched chain alkyl group having from 1 to 12 carbon atoms, preferably 1 to 4 carbon atoms, $R_3$ and $R_4$ are each independently selected from the group consisting of a straight or branched chain alkyl group having from 1 to 12 carbon atoms, preferably 1 to 8 carbon atoms, a cyclic alkyl group having from 5 to 10 carbon atoms, preferably 5 to 6, a 1 to 4 carbon atom alkyl substituted or unsubstituted benzyl group and an allyl group, $R_6$ is a straight or branched chain alkyl group having from 1 to 18 carbon atoms, preferably 1 to 8 carbon atoms, a cyclic alkyl group having from 5 to 10 carbon atoms, preferably 5 to 6 carbon atoms, a 1 to 4 carbon atom alkyl substituted or unsubstituted benzyl group or an allyl group and $A^{\ominus}$ represents an anion which provides electrical neutrality and may be selected from halides such as chloride, bromide, fluoride and iodide, sulfate, nitrate, alkyl and dialkyl sulfates such as methyl sulfate and ethyl sulfate, carbonate, carboxylate such as acetate including fatty acid carboxylates such as stearate and the like. Chloride, methyl sulfate and ethyl sulfate are particularly preferred anions.

The alkoxylated quaternary ammonium compounds are prepared by quaternization of an alkoxylated tertiary amine of the general formula:

$$RO(R_1)_xCH_2\overset{R_2}{\overset{|}{C}}HNR_3R_4$$

wherein R, $R_1$, $R_2$, $R_3$, $R_4$ and X are as herein above described, with an alkylating agent designated herein as $R_6A$ wherein $R_6$ and $A^{\ominus}$ are as hereinabove described.

A general postulated equation for the reaction to prepare the alkoxylated quaternary ammonium compounds of the present invention may be represented as follows with R, $R_1$, $R_2$, $R_3$, $R_4$, $R_6$, X and A as above described.

$$\underset{\text{alkoxylated tertiary amine}}{RO(R_1)_xCH_2\overset{R_2}{\overset{|}{C}}HNR_3R_4} + \underset{\text{alkylating agent}}{R_6A} \xrightarrow[\Delta + P]{} \underset{\text{quaternary ammonium compound}}{\left[RO(R_1)_xCH_2\overset{R_2}{\overset{|}{C}}H\overset{\oplus}{N}\ R_3R_4R_6\right]A^{\ominus}}$$

A wide variety of alkoxylated quaternary ammonium compounds can be prepared according to the invention.

The reaction may be carried out at temperatures in the range of from about 25° to 150°C, preferably from 60°C to 100°C with temperatures of approximately 70°C more preferred, and pressures of from about 1 atmosphere to 200 psig, preferably 50 to 100 psig with 60 psig most preferred. The ratio of total amine (whether pure alkoxylated tertiary amine or a tertiary amine containing some alkoxylated secondary amine $RO(R_1)_xCH_2\overset{R_2}{\overset{|}{C}}HNR_3H$ and alkoxylated primary amine $RO(R_1)_xCH_2\overset{R_2}{\overset{|}{C}}HNH_2$ impurities) to alkylating agent $R_6A$ is 1:3 preferably 1:1.

When the alkoxylated tertiary amine to be quaternized does contain secondary and/or primary amine impurities the quaternization reaction is generally carried out in the presence of from 1 to 50, preferably from 2 to 5 wt. % based on the total reaction mixture of an organic base material such as an alkaline earth metal or alkali metal hydroxide, carbonate, bicarbonate, etc. which acts as a scavenger for the acid produced during the reaction and helps force the reaction to completion.

If desired, the quaternization reaction may be carried out in the presence of a suitable inert solvent, such as water, alcohols, for example, methanol, ethanol, isopropanol, etc, ethers, for example diethyl ether, tetrahydrofuran, aromatic hydrocarbons, for example, benzene, toluene, xylene etc., as well as the alkanes, for example, hexane, heptane, pentane, etc.

The alkoxylated tertiary amines employed to prepare the alkoxylated quaternary ammonium compounds of the present invention may be obtained by amination of an oxyalkylated alcohol with a secondary amine in the presence of an amination catalyst as is described in the above-noted co-pending application.

The oxyalkylation reaction is conducted by methods well known in the art by reacting an appropriate alcohol such as, for example, methanol, etc. or the short chain plasticizer range linear Ziegler type alcohols containing various mixtures of $C_6$ through $C_{10}$ alcohols with an alkylene

oxide, such as propylene oxide in the presence of an alkali metal or alkaline earth catalyst as taught, for example in U. S. Patent Nos. 2,508,035; 2,617,830; 2,671,115 and 3,382,285 and incorporated herein by reference. Typical of such Ziegler alcohols, which are also employed in the instant invention, are the "EPAL 810 or 610" and "ALFOL 810 or 610" types which are mixtures of predominately $C_8$ and $C_{10}$ straight chain alcohols sold commercially, for example, by the Ethyl Corporation and the Continental Oil Company respectively.

The alcohols which may be reacted with the alkylene oxides to prepare the oxyalkylated alcohols for further processing include any aliphatic or branched monohydric alcohol containing from 1 to 11 carbon atoms, cyclic alcohols, aryl alcohols as well as aralkyl and alkaryl alcohols as defined by R in the above formula. Representative alcohols include, for example, methyl, ethyl, n-,iso-, sec-, and tert-butyl, amyl, hexyl, octyl, nonyl, n- and isopropyl, decyl, and benzyl alcohols, cyclohexanol, 2-ethyl hexanol, methyl cyclohexanol, 3-methyl butanol, 1-heptanol and the like or mixtures thereof.

The alkylene oxides which may be employed to prepare the oxyalkylated alcohols include, for example, ethylene and propylene oxide and the higher alkylene oxides, i.e., one containing from 3 to 12 carbon atoms. Preferred among the oxides is propylene oxide. Illustrative higher alkylene oxides include, for example, 1,2-epoxybutane, 1,2-epoxypentane, 5-methyl-1,2-epoxyheptane, 1,2-epoxyoctane, 2,4,4-trimethyl-2,3-epoxypentane, 1,2-epoxynonane, 1,2-epoxydecane, 1,2-epoxydodecane, and the like or mixtures thereof.

In general, the oxyalkylated alcohol formation with, for example propylene oxide, is carried out at temperatures in the range of from about 70°C to 150°C preferably 90°C to 100°C under moderately elevated pressures of from about atmospheric to 500 psig, preferably from 50 to 100 psig, and in the presence of between about 0.01 to 1, preferably 0.2 to 0.3 weight % of total reactants of an alkaline-reacting material or catalyst such as sodium, potassium, calcium, barium and strontium hydroxides. In general a controlled amount of an alkylene oxide or admixture thereof is slowly contacted with the alcohol reactant for a period ranging up to about 20 hours in an amount sufficient to prepare the desired oxyalkylated reaction product mixture. On completion of the oxyalkylation reaction, the crude reaction product containing the catalyst is normally treated with an inorganic acid or acid-forming material to neutralize the reaction product after which is filtered to produce a finished oxyalkylated product.

If desired the oxyalkylation of the alcohol may be carried out in the presence of a suitable inert solvent such as toluene, benzene, xylene, tetrahydrofuran, heptane, hexane, pentane, octane, etc. which are free of water.

Preparation of the alkoxylated tertiary amine involves the novel process of aminating the oxyalkylated alcohol with a secondary amine in the presence of from about 0.5 to 20 weight %, preferably 2.5 to 10 weight % of amination catalyst based on the total reaction mixture. Any amination catalyst known in the art may be employed, such as, for example, Raney Nickel, supported nobel metals, and catalysts containing Cu, Cr and promoters such as

nickel, however, catalysts containing CuO and $Cr_2O_3$ in various ratios, Cu-Cr supported on magnesium aluminate spinel and ruthenium on activated carbon are preferred catalysts.

The secondary amines which may be used to aminate the oxyalkylated alcohol include, for example, dimethylamine, diethylamine, di-n-propylamine, di-i-propylamine, di-n-butylamine, di-n-amylamine, di-n-hexylamine, di-n-octylamine, di-n-nonylamine, di-n-decylamine, di-i-octylamine, di-benzylamine, di(methylbenzyl)amine, di(ethylbenzyl)amine, dicyclohexylamine, dicyclopentylamine, and diallylamine.

The amination reaction of oxyalkylated alcohols employing second amines is conducted at a temperature within the range of from about 150°C to 325°C with a preferred range being from 175°C to 275°C. The pressure may be varied from about 50 psig to 3000 psig with the preferred range being 100 psig to 250 psig.

Although not required, solvents, if desired, which are chemically inert to the components of the reaction system may be employed in the reductive amination reaction. Suitable solvents include, for example, heptane, pentane, cyclohexane, benzene, toluene, xylene, etc.

The following Examples are provided to illustrate the present invention in accordance with the principles of this invention but are not to be construed as limiting the invention in any way except as indicated by the appended claims.

EXAMPLE 1

1000 grams of a mixture containing 92 weight % propoxylated tertiary amine prepared from "Epal 610"* to give $C_6-C_{10}-O(CH_2\overset{CH_3}{\overset{|}{C}}HO)_{11}CH_2\overset{CH_3}{\overset{|}{C}}HN(CH_3)_2$, 7.5 weight % propoxylated secondary amine $C_6-C_{10}-O(CH_2\overset{CH_3}{\overset{|}{C}}HO)_{11}CH_2\overset{CH_3}{\overset{|}{C}}HNHCH_3$ and 0.5 weight % propoxylated primary amine $C_6-C^{10}-O(CH_2\overset{CH_3}{\overset{|}{C}}HO)_{11}CH_2\overset{CH_3}{\overset{|}{C}}HNH_2$ was heated with 68.5 grams methyl chloride and 7.96 grams sodium hydroxide (50 weight % aqueous) in 250 grams isopropanol for six hours at 70°C. in a glass lined one gallon autoclave capable of withstanding 150 psig. After cooling and venting the pH was adjusted to 7.0 by adding a 50% HCl 10 to 1 dilution dropwise followed by filtration to remove solid sodium chloride. The solvent was removed under vacuum to give by $^{13}C$ NMR analysis a 99% isolated yield of 98% pure

$$\left[C_6-C_{10}-O(CH_2\overset{CH_3}{\overset{|}{C}}HO)_{11}CH_2\overset{CH_3}{\overset{|\,\oplus}{C}}HN(CH_3)_3\right]Cl^{\ominus}$$

alkoxylated quaternary ammonium compound. *"Epal 610" sold commercially by the Ethyl Corporation is a Ziegler type mixture of generally straight chain alcohols containing about 3.6% hexanol, 40.0% octanol and 55.4% decanol.

EXAMPLE 2

100 grams of a mixture containing 95 wt.% butoxylated tertiary amine prepared from "Epal 810"* to give $C_8-C_{10}-O\ (CH_2\overset{CH_2CH_3}{\overset{|}{C}}HO)_4CH_2\overset{CH_2CH_3}{\overset{|}{C}}HN(CH_2CH_3)_2$ and 5 wt.%

butoxylated secondary amine

$C_8-C_{10}-O\ (CH_2\overset{CH_2CH_3}{\overset{|}{C}}HO\ )_4\ CH_2\overset{CH_2CH_3}{\overset{|}{C}}HNH\ (CH_2CH_3)$ was heated with 38.16 grams octyl bromide and 3.0 grams solid sodium carbonate in 50 grams isopropanol for 6 hours at 70°C in a 500 ml, 3 neck reaction flask equipped with a reflux condenser and pot thermometer. After cooling and filtration the reaction mixture was stripped under vacuum to give by $^{13}C$ NMR analysis a 99%

yield of 95% $\left[ C_8-C_{10}-O(CH_2\overset{CH_2CH_3}{\overset{|}{C}}HO)_4CH_2\overset{CH_2CH_3}{\overset{|\ \oplus}{C}}HN(CH_2CH_3)_2\ \text{octyl} \right] Br^{\ominus}$

and 5% $\left[ C_8-C_{10}-O(CH_2\overset{CH_2CH_3}{\overset{|}{C}}HO)_4CH_2\overset{CH_2CH_3}{\overset{|\ \oplus}{C}}HN(CH_2CH_3)(\text{octyl})_2 \right] Br^{\ominus}$

**"Epal 810" sold commercially by the Ethyl Corporation is a Ziegler type mixture of generally straight chain alcohols containing about 0.3% hexanol, 44.3% octanol and 54.5% decanol.

EXAMPLE 3

100 grams of a mixture containing 94 wt.% propoxylated-ethoxylated tertiary amine prepared from "Epal 810" to give $C_8-C_{10}-O(CH_2\overset{CH_3}{\overset{|}{C}}HO)_8(CH_2CH_2O)_3CH_2CH_2N(CH_3)_2$ and 6 wt.% propoxylated-ethoxylated secondary amine $C_8-C_{10}-O(CH_2\overset{CH_3}{\overset{|}{C}}HO)_8(CH_2CH_2O)_3CH_2CH_2NH(CH_3)$ was reacted with 8.0 grams glacial acetic acid in the absence of added solvent for one hour at 50°C. The reaction mixture was stripped under vacuum to remove any unreacted acetic acid,

$^{13}C$ NMR analysis indicated a 98% yield of a liquid 94/6% mixture of

$$\left[C_8\text{-}C_{10}\text{-}O(CH_2\overset{\overset{CH_3}{|}}{C}HO)_8(CH_2CH_2O)_3CH_2CH_2\overset{\oplus}{N}(CH_3)_2H\right]\overset{\overset{O}{\|}}{O}\!\!\!\!\overset{\ominus}{\phantom{O}}CCH_3 \text{ and } \left[C_8\text{-}C_{10}\text{-}O(CH_2\overset{\overset{CH_3}{|}}{C}HO)_8(CH_2CH_2O)_3CH_2CH_2\overset{\oplus}{N}(CH_3)H_2\right]\overset{\overset{O}{\|}}{O}\!\!\!\!\overset{\ominus}{\phantom{O}}CCH_3$$

## EXAMPLE 4

100 grams of a mixture containing 95 wt.% propoxylated tertiary amine $CH_3(CH_2)_3CHC_2H_5CH_2O(CH_2\overset{\overset{CH_3}{|}}{C}HO)_9CH_2\overset{\overset{CH_3}{|}}{C}HN(CH_3)_2$ and 5 wt.% propoxylated secondary amine $CH_3(CH_2)_3CHC_2H_5CH_2O(CH_2\overset{\overset{CH_3}{|}}{C}HO)_9CH_2\overset{\overset{CH_3}{|}}{C}HN(CH_3)H$ was heated with a slight excess of dimethyl sulfate (18.0 grams) for six hours at 75°C in a stirred reaction flask. After cooling the reaction mixture was stripped under vacuum. $^{13}C$ NMR analysis indicated a 97.5% yield of

$$\left[2\text{-ethylhexyl-}O(CH_2\overset{\overset{CH_3}{|}}{C}HO)_9CH_2\overset{\overset{CH_3}{|}}{C}H\overset{\oplus}{N}(CH_3)_3\right]CH_3SO_4^{\ominus}.$$

## EXAMPLES 5 to 39

In examples 5 to 39 which follow in table form, the quaternization procedure of Example 1 was repeated using various alkoxylated tertiary amine compounds and alkylating agents. The reactions were carried out in a one gallon glass lined autoclave for a period of from 4 to 8 hours at an appropriate temperature and pressure. $R_5$ is the cationic portion of the alkylating agent and A is the counterion (anion). The resulting quaternary ammonium

compounds were analyzed by $^{13}C$ NMR indicating that % conversions were all essentially quantitative, i.e. 98-100%.

The reactants and conditions are set forth in Table 1 according to the following equation.

$$RO(R_1)_xCH_2\overset{R_2}{\overset{|}{C}}HNR_3R_4 + R_6A \longrightarrow \left[ RO(R_1)_xCH_2\overset{R_2}{\overset{|}{C}}H\overset{\oplus}{N}R_3R_4R_5 \right] A^{\ominus}$$

wherein $R_1$, $R_1$, $R_2$, $R_3$, $R_4$, $R_5$, $R_6$, X and A are as hereinabove described.

**TABLE 1**

| Ex. No. | R | $R_5$ | $R_2$ | $R_3$ | $R_4$ | X | Temp (°C) | $R_6$ | $A^{\ominus}$ |
|---|---|---|---|---|---|---|---|---|---|
| 5 | $C_8$-$C_{10}$ | $CH_3$ | $CH_3$ | $CH_3$ | $CH_3$ | 7 | 70 | *$CH_3$ | Cl |
| 6 | $C_8$-$C_{10}$ | $CH_3$ | $CH_3$ | $CH_3$ | $CH_3$ | 7 | 70 | *$CH_3$ | Cl |
| 7 | $C_8$-$C_{10}$ | $CH_3$ | $CH_3$ | $CH_3$ | $CH_3$ | 7 | 70 | $CH_3$ | $CH_3SO_4$ |
| 8 | $C_8$-$C_{10}$ | $CH_3$ | $CH_3$ | $CH_3$ | $CH_3$ | 7 | 70 | OC | Br |
| 9 | $C_8$-$C_{10}$ | $CH_3$ | $CH_3$ | $CH_3$ | $CH_3$ | 7 | 65 | 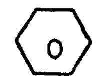 $CH_2$- | Cl |
| 10 | $C_8$-$C_{10}$ | $CH_3$ | $CH_3$ | $CH_3$ | $CH_3$ | 7 | 70 | $CH_2$-$CHCH_2$- | Cl |
| 11 | $C_8$-$C_{10}$ | $CH_3$ | $CH_3$ | $CH_3$ | $CH_3$ | 7 | 50 | H | $H_2BO_3$ |
| 12 | $C_8$-$C_{10}$ | $CH_3$ | $CH_3$ | $CH_3$ | $CH_3$ | 7 | 50 | H | $H_2PO_4$ |
| 13 | $C_8$-$C_{10}$ | $CH_3$ | $CH_3$ | $CH_3$ | $CH_3$ | 2 | 70 | H | $-OC(0)CH_3$ |
| 14 | $C_8$-$C_{10}$ | $CH_3$ | $CH_3$ | $CH_3$ | $CH_3$ | 4 | 80 | H | $-OC(0)(CH_2)_6CH_3$ |
| 15 | $C_8$-$C_{10}$ | $CH_3$ | $CH_3$ | $CH_3$ | $CH_3$ | 11 | 90 | H | $-OC(0)(CH_2)_{10}CH_3$ |
| 16 | $C_8$-$C_{10}$ | $CH_3$ | $CH_3$ | $CH_3$ | $CH_3$ | 15 | 70 | *$CH_3$ | Cl |
| 17 | $C_8$-$C_{10}$ | $CH_3$ | $CH_3$ | Ethyl | Ethyl | 7 | 70 | $CH_2CH_3$ | $CH_3CH_2$ |
| 18 | $C_8$-$C_{10}$ | $CH_3$ | $CH_3$ | 2-propyl | 2-propyl | 7 | 70 | $CH_2CH_2OH$ | OH |
| 19 | $C_8$-$C_{10}$ | $CH_3$ | $CH_3$ | n-butyl | n-butyl | 7 | 75 | *$CH_3$ | I |
| 20 | $C_8$-$C_{10}$ | $CH_3$ | $CH_3$ | n-octyl | n-octyl | 7 | 75 | *$CH_3$ | Cl |
| 21 | $C_8$-$C_{10}$ | $CH_3$ | $CH_3$ | allyl | allyl | 7 | 60 | *$CH_3$ | Cl |
| 22 | $C_8$-$C_{10}$ | $CH_3$ | $CH_3$ | benzyl | benzyl | 7 | 50 | *$CH_3$ | Cl |
| 23 | $C_8$-$C_{10}$ | $CH_3$ | $CH_3$ | $CH_3$ | $CH_3$ | 24 | 75 | H | Cl |

TABLE 1 (Cont'd)

| Ex. No. | R | $R_5$ | $R_2$ | $R_3$ | $R_4$ | X | Temp (°C) | $R_6$ | $A^{\ominus}$ |
|---|---|---|---|---|---|---|---|---|---|
| 24 | octyl | $CH_3$ | $CH_3$ | $CH_3$ | $CH_3$ | 19 | 70 | $*CH_3$ | Cl |
| 25 | decyl | $CH_3$ | $CH_3$ | $CH_3$ | $CH_3$ | 9 | 70 | $CH_3$ | Cl |
| 26 | $C_7$-$C_{11}$ | $CH_3$ | $CH_3$ | $CH_3$ | $CH_3$ | 7 | 70 | $*CH_3$ | Cl |
| 27 | 2-ethylhexyl | $CH_3$ | $CH_3$ | $CH_3$ | $CH_3$ | 11 | 70 | $CH_2CH_3$ | $CH_3CH_2S$ |
| 28 | hexyl | $CH_3$ | $CH_3$ | $CH_3$ | $CH_3$ | 19 | 60 | $CH_2CH_2OH$ | OH |
| 29 | 2-ethylhexyl | $CH_3$ | $CH_3$ | $CH_3$ | $CH_3$ | 4 | 60 | 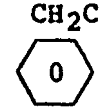 $CH_2-$ | Cl |
| 30 | $C_8$-$C_{10}$ | $CH_3$ | $CH_3$ | $CH_3$ | $CH_3$ | 7 | 90 | $*CH_2CH_3$ | Cl |
| 31 | $C_8$-$C_{10}$ | $CH_3$ | $CH_3$ | $CH_3$ | $CH_3$ | 7 | 90 | Butyl | Cl |
| 32 | $C_8$-$C_{10}$ | $CH_3$ | $CH_3$ | $CH_3$ | $CH_3$ | 7 | 90 | Octyl | Cl |
| 33 | $C_8$-$C_{10}$ | $CH_3$ | $CH_3$ | $CH_3$ | $CH_3$ | 7 | 70 | dodecyl | Cl |
| 34 | $C_8$-$C_{10}$ | $CH_3$ | ethyl | $CH_3$ | $CH_3$ | 8 | 70 | $*CH_3$ | Cl |
| 35 | $C_8$-$C_{10}$ | $8CH_3+3H^{(1)}$ (Random) | H | $CH_3$ | $CH_3$ | 11 | 70 | $*CH_3$ | Cl |
| 36 | $C_8$-$C_{10}$ | $4H_2+7CH_3$ (block) | $CH_3$ | $CH_3$ | $CH_3$ | 11 | 70 | $*CH_3$ | Cl |
| 37 | $C_8$-$C_{10}$ | ethyl | ethyl | $CH_3$ | $CH_3$ | 2 | 70 | $*CH_3$ | Cl |
| 38 | $C_8$-$C_{10}$ | 1 butyl $2CH_3^{(1)}$ (block) | $CH_3$ | $CH_3$ | $CH_3$ | 3 | 70 | $*CH_3$ | Cl |
| 39 | $C_8$-$C_{10}$ | 1 octyl $2CH_3^{(1)}$ (block) | $CH_3$ | $CH_3$ | $CH_3$ | 3 | 70 | $*CH_3$ | Cl |

* Carried out under 100 psig pressure all other examples at atmospheric pressure.

(1) In these examples total ($R_1$) units are represented.

CLAIMS

1. An alkoxylated quaternary ammonium compound of the formula

$$\left[RO(R_1)_xCH_2\overset{\overset{\displaystyle R_2}{|}}{C}H\overset{\oplus}{N}R_3R_4R_6\right] \qquad A^{\ominus}$$

or a mixture of such compounds, wherein R is a straight or branched chain alkyl group having from 1 to 11 carbon atoms, a cyclic alkyl group having from 5 to 10 carbon atoms, an aryl group having up to 12 carbon atoms or an aralkyl or alkaryl group having up to 18 carbon atoms, each $R_1$ is a unit of the formula:

$$-(CH_2\overset{\overset{\displaystyle R_5}{|}}{C}HO)-$$

wherein in each unit $R_5$ is independently selected from hydrogen and straight and branched chain alkyl groups having from 1 to 12 carbon atoms, x is an integer of from 0 to 40, $R_2$ is hydrogen or a straight or branched chain alkyl group having from 1 to 12 carbon atoms, $R_3$ and $R_4$ are each independently selected from straight and branched chain alkyl groups having from 1 to 12 carbon atoms, cyclic alkyl groups having from 5 to 10 carbon atoms, 1 to 4 carbon atom alkyl substituted or unsubstituted benzyl groups and allyl, $R_6$ is a straight or branched chain alkyl group having from 1 to 18 carbon atoms, a cyclic alkyl group having from 5 to 10 carbon atoms, a 1 to 4 carbon atom substituted or unsubstituted benzyl group or an allyl group and A is an anion.

2. An alkoxylated quaternary ammonium compound as claimed in claim 1, or a mixture of such compunds, wherein the anion A is selected from halide, sulfate, nitrate, alkyl sulfate, carbonate and carboxylate.

3. An alkoxylated quaternary ammonium compound, or mixture of such compounds, as claimed in claim 2, wherein A is acetate, chloride, methyl sulfate or ethyl sulfate.

4. An alkoxylated quaternary ammonium compound, or mixture of compounds, as claimed in any one of claims 1 to 3 wherein R is a straight chain alkyl group having from 6 to 10 carbon atoms.

5. An alkoxylated quaternary ammonium compound or mixture of compounds, as claimed in any one of claims 1 to 3 wherein R is $C_7$-$C_{11}$.

6. An alkoxylated quaternary ammonium compound or mixture of compounds, as claimed in any one of claims 1 to 5 wherein $R_5$ is $CH_3$-, $CH_3CH_2$- or $CH_3CH_2CH_2$-.

7. An alkoxylated quaternary ammonium compound or mixture of compounds, as claimed in any one of claims 1 to 6 which contains $R_1$ units which are different and are in random configuration.

8. An alkoxylated quaternary ammonium compound, or mixture of compounds, as claimed in any one of claims 1 to 6 which contains $R_1$ units which are different and are in block configuration.

9. An alkoxylated quaternary ammonium compound or mixture of compounds, as claimed in any one of claims 1 to 8 wherein $R_3$ and $R_4$ are methyl.

10. An alkoxylated quaternary ammonium compound or mixture of compounds as claimed in any one of claims 1 to 9 wherein $R_6$ is methyl.

11. An alkoxylated quaternary ammonium compound or mixture of compounds as claimed in any one of claims 1 to 10 wherein x is 7.

12. An alkoxylated quaternary ammonium compound of the formula:

$$\left[ RO(R_1)_x CH_2 CH\overset{\overset{\displaystyle R_2}{|}}{N}{}^{\oplus} R_3 R_4 R_6 \right] \quad A^{\ominus}$$

wherein R is a $CH_3(CH_2)_3CHC_2H_5CH_2$ group, $R_1$ is $\leftarrow CH_2\overset{\overset{\displaystyle CH_3}{|}}{C}HO \rightarrow$, x is 7, $R_2$, $R_3$, $R_4$ and $R_6$ are each a $CH_3$ group and A is chloride.

European Patent Office

# EUROPEAN SEARCH REPORT

0182669
Application number

EP 85308489.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE - A - 2 250 133 (CASSELLA FARBWERKE)<br>* Examples 2,3,4,6; claim 1; page 4, line 5 - page 6, line 18 *<br>-- | 1-3,9 | C 07 C 93/04 |
| X | DE - A1 - 3 126 522 (BASF)<br>* Example 1 *<br>-- | 1-3,9 | |
| X | N. SCHÖNFELDT "Grenzflächenaktive Äthylenoxid-Addukte" 1976<br>WISSENSCHAFTLICHE VERLAGSGESELL-SCHAFT MBH, Stuttgart<br>pages 972-982<br>* Page 973, line 39; page 978, lines 5-8 *<br>-- | 1-3,9 | |
| | | | **TECHNICAL FIELDS SEARCHED (Int. Cl.4)** |
| X | BEILSTEINS "Handbuch der Organischen Chemie", vol. 4, supplement IV, 1979<br>SPRINGER VERLAG, Berlin<br>page 1445<br>* Page 1445, lines 1-4 *<br>-- | 1-3,9 | C 07 C 93/00 |
| X | BEILSTEINS "Handbuch der Organischen Chemie", vol. 4, supplement I, 1929<br>VERLAG VON JULIUS SPRINGER, Berlin<br>page 427<br>* Page 427, lines 52-59 *<br>-- | 1-3,9 | |

The present search report has been drawn up for all claims

| Place of search | Date of completion of the search | Examiner |
|---|---|---|
| VIENNA | 27-01-1986 | KÖRBER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0182669

Application number



EP 85308489.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB - A - 1 084 134 (MARCHON PRODUCTS)<br>* Claims 1,7 *<br>---- | 1-3 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 27-01-1986 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document